(19) Europäisches Patentamt
European Patent Office
Office européen des brevets

(11) **EP 2 638 894 B1**

(12) **EUROPÄISCHE PATENTSCHRIFT**

(45) Veröffentlichungstag und Bekanntmachung des Hinweises auf die Patenterteilung:
**14.09.2016  Patentblatt 2016/37**

(21) Anmeldenummer: **13001361.8**

(22) Anmeldetag: **18.03.2013**

(51) Int Cl.:
*A61K 9/00* *(2006.01)*       *A61K 47/24* *(2006.01)*
*A61K 31/192* *(2006.01)*   *A61K 31/196* *(2006.01)*
*A61K 9/12* *(2006.01)*       *A61P 29/00* *(2006.01)*

(54) **Pharmazeutische Zusammensetzung mit Phospholipid**

Pharmaceutical composition containing phospholipid

Composition pharmaceutique comprenant un phospholipide

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.03.2013   DE 102013004199**
**15.05.2012   DE 102012009575**

(43) Veröffentlichungstag der Anmeldung:
**18.09.2013   Patentblatt 2013/38**

(73) Patentinhaber: **MIKA Pharma Gesellschaft für die Entwicklung und**
**Vermarktung pharmazeutischer Produkte mbH**
**67346 Speyer (DE)**

(72) Erfinder: **Seigfried, Bernd G.**
**67117 Limburgerhof (DE)**

(74) Vertreter: **Lau-Loskill, Philipp**
**Am Büschgen 33**
**41189 Mönchengladbach (DE)**

(56) Entgegenhaltungen:
**EP-A1- 0 704 206       EP-A2- 0 521 398**
**DE-A1-102010 027 315**

- **GUO Z ET AL: "Enzymatic modification of phospholipids for functional applications and human nutrition", BIOTECHNOLOGY ADVANCES, ELSEVIER PUBLISHING, BARKING, GB, Bd. 23, Nr. 3, 1. Mai 2005 (2005-05-01), Seiten 203-259, XP027719397, ISSN: 0734-9750 [gefunden am 2005-05-01]**

**Beschreibung**

Die vorliegende Erfindung betrifft eine pharmazeutische Zusammensetzung zur Anwendung beim Mensch und Tier mit den Merkmalen des Oberbegriffs des Patentanspruchs 1.

Pharmazeutische Zusammensetzungen zur Anwendung beim Mensch und Tier, die mindestens einen systemisch und/oder lokal wirkenden, topisch applizierbaren Wirkstoff aufweisen, sind seit langem bekannt.

So beschreibt beispielsweise die EP 0 704 206 A eine derartige pharmazeutische Zusammensetzung, die als Flüssigkeit vorliegt und die als Tröpfchen topisch aufsprühbar ist. Ebenso bekannt ist aus der DE 10 2010 027 315 eine solche pharmazeutische Zusammensetzung, die als Flüssigkeit vorliegt und die als Schaum beim Mensch oder Tier topisch appliziert wird, wobei beide bekannten Zusammensetzungen übereinstimmend ein Phospholipid aufweisen, das Phosphatidylcholin in einer Konzentration von wenigstens 60 Gew.%, bezogen auf den Gesamtgehalt an Phospholipid, enthalten. Der Ölgehalt dieses Phospholipids wird weder in der EP 0 704 206 noch in der DE 10 2010 027 315 A quantifiziert. Zwar nennt die EP 0 704 206 A für den dort angesprochenen phospholipidischen Gelbildner eine Konzentration von 9 Gew.% an anderen, nicht näher analysierten üblichen Begleitlipiden, läßt jedoch offen, wie diese Begleitlipide bestimmt werden, während die DE 10 2010 027 315 A1 zwar ölartige Bestandteile beschreibt, aber ebenfalls nicht darlegt, wie diese ölartigen Bestandteile quantitativ bestimmt werden. Desweiteren beschreibt die EP 0 521 398 A2 an einer einzigen Stelle, nämlich auf Seite 5, Zeilen 25 bis 32, eine Ölkonzentration eines Phospholipids, das 94,2 Gew.% Phosphatidylcholin und 0,8 Gew.% eines Triglycerids aufweist, während eine Beschleunigung der Penetration oder Permeation des Wirkstoffes durch die Zellmembran, bewirkt durch das zuvor quantifizierte Phospholipid, nicht erwähnt ist. Schließlich ist aus der Publikation "Enzymatic modification of phospholipids for functional applications and human nutrition"; Zheng Guo et al in Biotechnology Advances 23 (2005) 203-259 eine Reihe von Phospholipiden zu entnehmen, deren Ölkonzentrationen entweder bei 1 % oder bei 21 %, 25 % und 35 % liegen.

Der vorliegenden Erfindung liegt die Aufgabe zugrunde, eine pharmazeutische Zusammensetzung der angegebenen Art zur Verfügung zu stellen, die ein besonders hohes Permeationsvermögen für pharmazeutische Wirkstoffe besitzt.

Diese Aufgabe wird erfindungsgemäß durch eine Zusammensetzung mit den kennzeichnenden Merkmalen des Patentanspruchs 1 gelöst.

Die erfindungsgemäße pharmazeutische Zusammensetzung, die zur topischen Anwendung beim Menschen oder beim Tier verwendet, wird, weist mindestens einen, systemisch und/oder lokal wirkenden, topisch applizierbaren pharmazeutischen Wirkstoff auf, der nachfolgend auch kurz als Wirkstoff bezeichnet wird. Desweiteren ist in der erfindungsgemäßen Zusammensetzung mindestens ein, den Transport des Wirkstoffes bei einer topischen Applikation der erfindungsgemäßen Zusammensetzung durch die Zellmembrane verbesserndes Phospholipid enthalten, wobei das Phospholipid eine Konzentration an Phosphatidylcholin von wenigstens 60 Gew.%, bezogen auf das Gesamtgewicht des Phospholipids (Trockengewicht), enthält. Die erfindungsgemäße pharmazeutische Zusammensetzung besitzt eine solche flüssige Konsistenz, daß sie als Tröpfchen oder Nebel über geeignete, handelsübliche Applikatoren, so z.B. über entsprechende Applikatoren, die von der Firma Calmar/MeadWestvaco (Keltec) hergestellt und vertrieben werden, aufsprühbar und somit topisch applizierbar ist. Erfindungsgemäß ist in der beanspruchten Zusammensetzung ein solches Phospholipid enthalten, das neben wenigstens 60 Gew.% Phosphatidylcholin desweiteren Öl in einer Konzentration zwischen 4,8 Gew.% und 2 Gew.%, bezogen auf das Trocken-Gesamtgewicht des Phospholipids, aufweist. Hierbei erfolgt die quantitative Bestimmung dieses Öls, das in der erfindungsgemäßen Zusammensetzung in dem Phospholipid enthalten ist, nach der Analysenmethode, wie sie nachfolgend zu Beginn der Ausführungsbeispiele unter der Überschrift "quantitative Bestimmung des im Phospholipid enthaltenen Öls" exakt beschrieben ist.

Die erfindungsgemäße Zusammensetzung weist eine Reihe von Vorteilen auf. So ist zunächst festzuhalten, daß die erfindungsgemäße Zusammensetzung eine verbesserte pharmazeutische Wirksamkeit besitzt, was darauf zurückgeführt wird, daß durch Auswahl des zuvor beschriebenen speziellen Phospholipids, das sich einerseits durch eine Mindestkonzentration von 60 Gew.% Phosphatidylcholin und andererseits durch eine begrenzte Konzentration an Öl zwischen 4,8 Gew.% und 2 Gew.%, auszeichnet, eine beschleunigte Penetration und insbesondere eine beschleunigte Permeation der systemisch und/oder lokal wirkenden pharmazeutischen Wirkstoffe herbeiführt, so daß dementsprechend diese Wirkstoffe in höheren Konzentrationen und/oder schneller bei der topischen Applikation der pharmazeutischen Zusammensetzung an den jeweiligen Wirkort gelangen. Desweiteren konnte überraschend festgestellt werden, daß durch Begrenzung der Ölkonzentration in den in der erfindungsgemäßen Zusammensetzung enthaltenen Phospholipiden eine Verbesserung der Lagerstabilität der erfindungsgemäßen Zusammensetzung herbeigeführt wird, was darauf zurückgeführt wird, daß derartige, in den Phospholipiden üblicherweise vorhandenen Ölen oftmals eine hohe Konzentration an ungesättigten Doppelbindungen aufweisen, die eine relativ hohe Oxidationsempfindlichkeit bei Lagerung in einer Luftatmosphäre besitzen. Sollten derartige Oxidationsvorgänge verstärkt ablaufen, können diese, so nach den Vorstellungen des Erfinders der vorliegenden Anmeldung, dazu führen, daß unerwünschte Oxidationsprodukte entstehen, die ihrerseits insbesondere bei oxidationsempfindlichen Wirkstoffen den beschleunigten Zerfall des Wirkstoffes und/oder des Phospholipids katalysieren und/oder herbeiführen, was eine verringerte Lagerstabilität und/oder eine reduzierte pharmazeutische Wirksamkeit verursachen kann. Treten die zuvor beschriebenen Oxidationsvorgänge des im Phospholipid vorhandenen Öls auf und bewirken oder katalysieren diese Ölabbauprodukte den Abbau des Phospholipids, so führen bereits geringe Spuren von Phospholipidabbauprodukten zu einer unangenehmen und unerwünschten Geruchsentwicklung in der erfindungsgemäßen Zusammensetzung, die um so stärker wird, je länger die erfindungsgemäße Zusammensetzung nach der ersten Benutzung gelagert wird. Diese Geruchsentwicklung hält wiederum den Patienten davon ab, die erfindungsgemäße Zusammensetzung in dem Umfang anzuwenden, wie er medizinisch indiziert und/oder notwendig ist.

Die zuvor angesprochene erhöhte Penetration und Permeation der erfindungsgemäßen Zusammensetzung wird nach Auffassung des Erfinders auch darauf zurückgeführt, daß in dem Öl insbesondere freie Fettsäuren, Sterole, Mono- und Diglyceride, Triglyceride, Tocopherol und/oder Fettsäureester sowie vergleichbare Substanzen enthalten sind, die die Permeation und/oder die Penetration des Wirkstoffes negativ beeinflussen können.

Wie bereits vorstehend bei der erfindungsgemäßen Zusammensetzung beschrieben, weist die erfindungsgemäße Zusammensetzung mindestens ein Phospholipid auf, wobei es sich vorzugsweise hierbei um ein Phospholipidgemisch handelt, das neben Phosphatidylcholin als Hauptbestandteil noch Lyso-Phosphatidylcholin, Phosphatidsäure, Phosphatidylethanolamin und/oder Phosphatidylinositol enthält.

Desweiteren deckt der in der vorliegenden Beschreibung verwendete Begriff "und/oder" sowohl additiv als auch alternativ die so verknüpften einzelnen Elemente einer Aufzählung ab, so daß diese Elemente wahlweise mit "und" bzw. mit "oder" verknüpft zu verstehen sind. Desweiteren umfassen die im Singular verwendeten Begriffe selbstverständlich auch den Plural.

Ferner ist festzuhalten, daß der in der vorliegenden Beschreibung verwendete Begriff Phospholipid selbstverständlich nicht nur ein einzelnes Phospholipid sondern auch ein Gemisch von Phospholipiden abdeckt, wobei das Phospholipid bzw. das Phospholipidgemisch natürlichen oder synthetischen Ursprungs sein kann.

Somit kann grundsätzlich in der erfindungsgemäßen Zusammensetzung jedes Phospholipid enthalten sein, sofern dieses Phospholipid die zuvor genannte Mindestkonzentration an Phosphatidylcholin von wenigstens 60 Gew.% sowie die vorstehend bei der erfindungsgemäßen Zusammensetzung aufgeführten Maximalkonzentrationen an Öl aufweist. Von daher sind auch solche Ausführungsformen der erfindungsgemäßen Zusammensetzung durch die vorliegende Beschreibung abgedeckt, bei denen das in der erfindungsgemäßen Zusammensetzung enthaltene Phospholipid bzw. Phospholipidgemisch ein synthetisches Phospholipid bzw. ein synthetisches Phospholipidgemisch ist. Vorzugsweise weist jedoch die erfindungsgemäße pharmazeutische Zusammensetzung ein solches Phospholipid bzw. Phospholipidgemisch auf, das aus pflanzlichen Bestandteilen, insbesondere aus Getreidesamen und/oder ölreichen Samen und vorzugsweise aus Sojabohnen oder Sonnenblumen isoliert ist.

Bei einer weiteren Ausführungsform der erfindungsgemäßen pharmazeutischen Zusammensetzung enthält diese ein solches Phospholipid, dessen Konzentration an Phosphatidylcholin mindestens 75 Gew.% beträgt, wobei eine ganz besonders bevorzugte Ausgestaltung der erfindungsgemäßen Zusammensetzung ein solches Phospholipid aufweist, bei dem die Konzentration an Phosphatidylcholin bei 78,1 Gew.% ± 3 Gew.% liegt. Die zuvor aufgeführten Konzentrationswerte des Phosphatidylcholins beziehen sich auf das Trockengewicht des Phospholipids.

Vorstehend ist bereits bei der Beschreibung der erfindungsgemäßen Zusammensetzung wiederholt darauf hingewiesen worden, daß das Phospholipid auch ein Phospholipidgemisch sein kann. Insbesondere weist das in der erfindungsgemäßen Zusammensetzung enthaltene Phospholipid eine Konzentration an Lyso-Phosphatidylcholin von weniger als 5,6 Gew.%, vorzugsweise eine Konzentration zwischen 5,5 Gew.% und 1,5 Gew.%, an Phosphatidylethanolamin von weniger als 5,2 Gew.%, vorzugsweise eine Konzentration zwischen 5,1 Gew.% und 2,3 Gew.%, und an Phosphatidsäure von weniger als 2,9 Gew.%, vorzugsweise eine Konzentration zwischen 2,5 Gew.% und 0,9 Gew.%, jeweils bezogen auf die Gesamtkonzentration an Phospholipid, auf. Ferner können in der erfindungsgemäßen Zusammensetzung Glycophospholipide, Sterine oder sonstige Begleitphospholipide, insbesondere sonstige Lyso-Phospholipide, vorzugsweise in geringen Konzentrationen, d.h. Konzentrationen im Bereich von etwa 1 Gew.% bis 2 Gew.%, enthalten sein.

Vorzugsweise liegt die erfindungsgemäße Zusammensetzung vor ihrer Applikation als flüssige Zusammensetzung vor, die als Nebel oder Tröpfchen appliziert wird. Hierbei enthält dann die erfindungsgemäße Zusammensetzung, abhängig von der gewünschten und/oder für die jeweilige Applikation erforderlichen Viskosität ein anorganisches oder organisches Lösungsmittel, wobei als anorganisches Lösungsmittel Wasser und als organisches Lösungsmittel ein physiologisch unbedenkliches Lösungsmittel in der erfindungsgemäßen Zusammensetzung enthalten ist.

Wasser im Sinne der vorliegenden Beschreibung umfaßt alle wäßrigen Systeme, die physiologisch unbedenklich und rechtlich zugelassen sind und deckt neben destilliertem Wasser, entionisiertem Wasser, hochreinem Wasser auch solche wäßrigen Systeme ab, die zur Korrektur des pH-Wertes entsprechende Puffersysteme enthalten oder auch Salze, insbesondere Kochsalz, aufweisen.

Als bevorzugte organische, physiologisch unbedenkliche Lösungsmittel enthält die erfindungsgemäße Zusammensetzung neben Wasser oder zusätzlich zu Wasser mindestens einen Alkohol, insbesondere Ethanol, Isopropanol, ein oder mehrere Glykole und/oder Glycerin. Üblicherweise wird bei Verwendung von Ethanol in der erfindungsgemäßen Zusammensetzung dessen Konzentration begrenzt, so insbesondere auf eine Konzentration von maximal 8 Gew.% und vorzugsweise auf eine Konzentration zwischen 2 Gew.% und 6 Gew.%, jeweils bezogen auf das Gesamtgewicht der erfindungsgemäßen Zusammensetzung.

Geeignete Glykole, die als organische Lösungsmittel bei speziellen Ausführungsformen in der erfindungsgemäßen Zusammensetzung vorgesehen sind, werden aus der Gruppe ausgewählt, die Propylenglykol, Butylenglykol, Pentylenglykol und Hexylenglykol umfaßt.

Eine weitere Ausführungsform der erfindungsgemäßen Zusammensetzung sieht vor, daß diese ein Lösungsmittelgemisch aus Wasser und mindestens einem Alkohol, vorzugsweise Isopropanol, enthält.

Abhängig von der erwünschten und/oder der für die jeweilige Applikationsform erforderlichen Viskosität, variiert die Konzentration des in der erfindungsgemäßen Zusammensetzung enthaltenen anorganischen und/oder organischen Lösungsmittels. Vorzugsweise liegt die Konzentration des Wassers in der flüssigen erfindungsgemäßen Zusammensetzung zwischen 50 Gew.% und 95 Gew.%, insbesondere zwischen 55 Gew.% und 75 Gew.%, und die Konzentration des mindestens einen organischen Lösungsmittels und vorzugsweise des Alkohols zwischen 5 Gew.% und 50 Gew.%, insbesondere zwischen 8 Gew.% und 25 Gew.%.

Bezüglich des in der erfindungsgemäßen Zusammensetzung enthaltenen mindestens einen pharmazeutischen Wirkstoffes ist allgemein festzuhalten, daß es sich hierbei um einen solchen pharmazeutischen Wirkstoff handelt, der bei einer topischen Applikation eine lokale und/oder systemische pharmazeutische Wirksamkeit besitzt. Insbesondere wird der in der erfindungsgemäßen Zusammensetzung enthaltene Wirkstoff aus der Gruppe ausgewählt, die Lokalanästhetika, Antiallergika, Dermatika, Wirkstoffe gegen grippale Infekte und Erkältungskrankheiten, Wirkstoffe zur Behandlung von Neuropathien, Wirkstoffe zur Behandlung von Durchblutungsstörungen, Chemotherapeutika, Chinin, Antimykotika, Antibiotika, Thalidomid, Serotonin, Eicosanoide, Analgetika, Antikonvulsiva, Nicht-steroidale Antirheumatika, Leukotriene, Leukotrienhemmer, Androgene, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren (einzel- und doppelsträngige DNA, einzel- und doppelsträngige RNA, snRNA, DNA-Oligonukleotide, RNA-Oligonukleotide) und Oligopeptide, Antipruriginosa, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Warzen oder Wunden, insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Antiparasitika, Ganglionär angreifende Substanzen, am Symphatikus angreifende Substanzen, am Parasymphatikus angreifende Substanzen, antibakteriell wirkende Pharmaka, Calciumantagonisten, Herz-/Kreislaufmittel, Antiasthmatika, Antitussiva, Expektorantien, Hepatika, Diuretika, Choleretika, Desinfektionsmittel, Spurenelemente, Antiinfektiva, Zytostatika, Antimetaboliten, Hormonantagonisten, Immunmodulatoren sowie Derivate und Salze der zuvor genannten Wirkstoffe umfaßt.

Abhängig von dem jeweiligen Wirkstoff und der Art der topischen Applikation variiert die Konzentration des Wirkstoffes in der erfindungsgemäßen Zusammensetzung und liegt zwischen 0,01 Gew.% und 15 Gew.%, bezogen auf das Gesamtgewicht der erfindungsgemäßen Zusammensetzung.

Besonders geeignete Ausführungsformen, die sich durch eine besonders hohe pharmazeutische Wirksamkeit bei einer topischen Applikation auszeichnet, enthalten als mindestens einen pharmazeutischen Wirkstoff ein Analgetikum, das aus der Gruppe ausgewählt ist, die Diclofenac, Ketoprofen und Ibuprofen umfaßt.

Wird bei der zuvor beschriebenen Ausführungsform als Wirkstoff Diclofenac ausgewählt, so weist vorteilhafterweise die erfindungsgemäße Zusammensetzung ein Diclofenac-Derivat und vorzugsweise ein Alkalisalz von Diclofenac auf, das insbesondere als Natriumsalz in der erfindungsgemäßen Zusammensetzung vorhanden ist.

Gute Ergebnisse lassen sich mit der topisch zu applizierenden erfindungsgemäßen Zusammensetzung, die als Wirkstoff Diclofenac enthält, dadurch erreichen, daß das Diclofenac und insbesondere das Alkalisalz von Diclofenac und vorzugsweise das Natriumsalz von Diclofenac in einer Konzentration zwischen 0,1 Gew.% und 10 Gew.%, vorzugsweise in einer Konzentration zwischen 1 Gew.% und 6 Gew.% und insbesondere zwischen 2 Gew.% und 4 Gew.%, in der Zusammensetzung enthalten ist.

Weist hingegen eine andere Ausführungsform der erfindungsgemäßen Zusammensetzung als mindestens einen Wirkstoff Ketoprofen auf, so variiert hierbei die Konzentration des Ketoprofens in der erfindungsgemäßen Zusammensetzung zwischen 5 Gew.% und 15 Gew.%, vorzugsweise zwischen 8 Gew.% und 12 Gew.%.

Bezüglich der Konzentration an Phospholipid, das in der erfindungsgemäßen Zusammensetzung enthalten ist, ist allgemein festzuhalten, daß sich diese Konzentration insbesondere danach richtet, welcher Wirkstoff in der erfindungsgemäßen Zusammensetzung enthalten ist, welche Erkrankung mit der erfindungsgemäßen Zusammensetzung topisch und/oder systemisch behandelt werden soll und welche Art der Applikation, sei es als Nebel oder Tröpfchen, ausgewählt wird. Insbesondere läßt sich durch die Variation der Konzentration des in der erfindungsgemäßen Zusammensetzung enthaltenen mindestens einen Phospholipids auch die Viskosität der flüssigen Zusammensetzung variieren, so daß dementsprechend eine erwünschte Größe der Nebeltröpfchen oder Tröpfchen eingestellt werden kann. Vorzugsweise weist die erfindungsgemäße Zusammensetzung das mindestens eine Phospholipid in einer Konzentration zwischen 0,5 Gew.% und 20 Gew.%, insbesondere in einer Konzentration zwischen 5 Gew.% und 13 Gew.%, auf.

In Weiterbildung der zuvor beschriebenen Ausführungsformen der erfindungsgemäßen Zusammensetzung weist diese insbesondere desweiteren mindestens einen Komplexbildner, insbesondere Ethylentetraessigsäure, mindestens eine Puffersubstanz, insbesondere einen Phosphatpuffer, mindestens ein Antioxidationsmittel, insbesondere Palmitoylascorbinsäure, mindestens einen Duftstoff und/oder ein Aroma auf.

Der in dem vorliegenden Text verwendete Begriff "topisch" umfaßt jede örtlich begrenzte äußerliche Anwendung der erfindungsgemäßen Zusammensetzung, insbesondere eine Applikation auf die Haut von Mensch und Tier. Hierbei deckt der Begriff Haut nicht nur die jeweils erkrankten Hautbereiche sondern auch gesunde Hautbereiche sowie alle zugänglichen Oberflächen des menschlichen oder tierischen Körpers ab, auf die die erfindungsgemäße Zusammensetzung zur lokalen Anwendung und/oder zur systemischen Anwendung appliziert werden kann, so insbesondere neben der eigentlichen Haut oder Kopfhaut auch Nägel, Haare, Zähne, Hufe oder die Schleimhaut in Mund, Nase, Vagina oder Vorhaut, die Bereiche des Ohrs und insbesondere die Bereiche des inneren Ohres, den Bereich des Darmausgangs und des Enddarmes, den Bereich der Augen, insbesondere den Bereich unter dem Augenlid, wie beispielsweise Bindehaut, Hornhaut und Tränensack zu nennen sind.

Die vorliegende Erfindung betrifft desweiteren die Verwendung von mindestens einem Phospholipid als Permeations- und/oder Penetrationsbeschleuniger in einer pharmazeutischen Zusammensetzung, wie diese zuvor als erfindungsgemäße pharmazeutische Zusammensetzung beschrieben ist und die bei Mensch und Tier angewendet wird und mindestens einen systemisch und/oder lokal wirkenden, topisch applizierbaren Wirkstoff aufweist. Um diesen Wirkstoff durch die Zellmembrane zu transportieren, sieht die erfindungsgemäße Verwendung vor, daß das Phospholipid, das Phosphatidylcholin in einer Konzentration von wenigstens 60 Gew.%, bezogen auf das Phospholipid, aufweist und desweiteren Öl in einer Konzentration von maximal 4,8 Gew.% bis 2 Gew.% enthält. Hierbei besitzt diese pharmazeutische Zusammensetzung eine solche flüssige Konsistenz, daß sie als Tröpfchen versprühbar ist.

Die erfindungsgemäße Verwendung beinhaltet alle die Vorteile analog oder identisch, wie sie zuvor ausgiebig bei der erfindungsgemäßen pharmazeutischen Zusammensetzung beschrieben sind, so daß zur Vermeidung von Wiederholungen hierauf ausdrücklich verwiesen wird. Insbesondere ist jedoch hervorzuheben, daß sich überraschend gezeigt hat, daß die Ölkonzentration einen entscheidenden Einfluß auf das Penetrations- und Permeationsverhalten des wenigstens einen Wirkstoffes hat, dahingehend, daß der Wirkstoff bei einer topischen Anwendung schneller in und/oder durch die jeweilige Barriere, so insbesondere durch die Haut, die Kopfhaut, das Fell, die Nägel, die Haare, die Zähne, die Hufe, die Schleimhaut in Mund, Nase, Vagina oder Vorhaut, durch die Bereiche des Ohres, der Darmausgangs und des Enddarms, den Bereich der Augen und vorzugsweise den Bereich unterhalb des Augenlids transportiert wird.

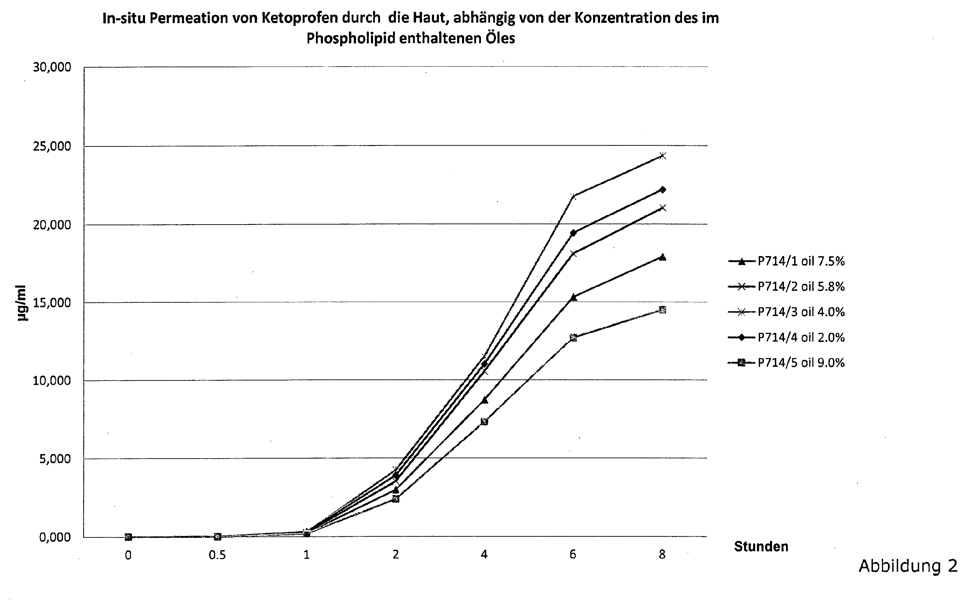

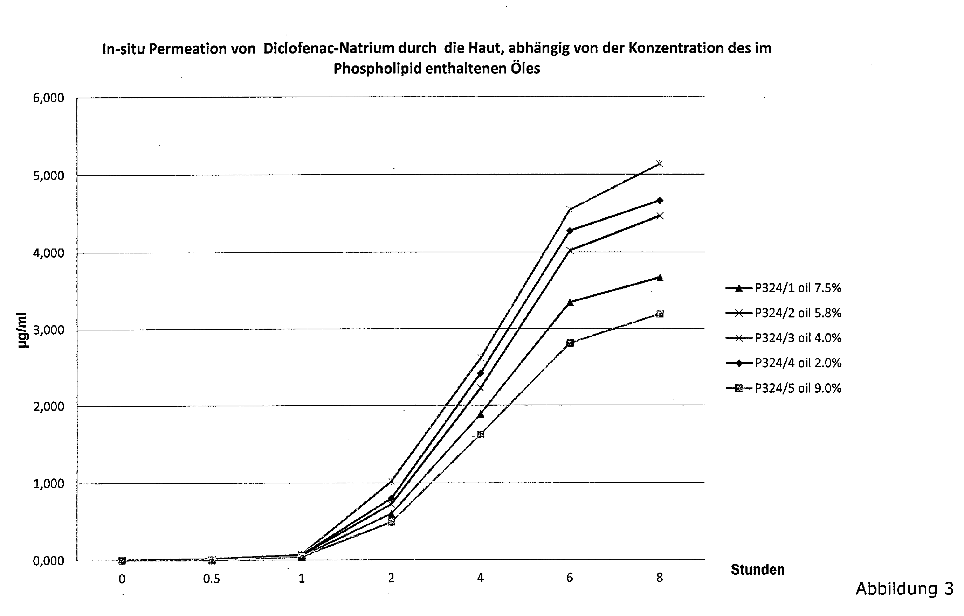

Am stärksten ist die erhöhte Penetrations- und Permeationsverbesserung dann festzustellen, wenn die Ölkonzentration im Phospholipid zwischen 4 Gew.% und 2 Gew.% variiert, wie dies die nachfolgend noch im Detail bei den Ausführungsbeispielen beschriebenen Abbildungen 2 und 3 augenfällig belegen.

Eine bevorzugte Weiterbildung der erfindungsgemäßen Verwendung sieht vor, daß hierbei das Phospholipid ein Phospholipidgemisch ist, das desweiteren neben Phosphatidylcholin noch Lyso-Phosphatidylcholin, Phosphatidsäure, Phosphatidylethanolamin und/oder Phosphatidylinositol aufweist.

Bevorzugt ist das bei der erfindungsgemäßen Verwendung eingesetzte Phospholipid ein aus pflanzlichen Bestandteilen, insbesondere aus Getreidesamen und/oder ölreichen Samen und vorzugsweise aus Sojabohnen oder Sonnenblumen, isoliertes Phospholipid.

Insbesondere dann, wenn bei der erfindungsgemäßen Verwendung das Phospholipid eine Konzentration an Phosphatidylcholin von mindestens 75 Gew.% und insbesondere 78,1 Gew.% $\pm$ 3 Gew.% aufweist, sind die zuvor beschriebenen Verbesserungen des Penetrations- und Permeationsvermögens weiter gesteigert, wobei sich diese und die folgenden Konzentrationsangaben auf das Trockengewicht des jeweiligen Phospholipids beziehen.

Besonders geeignet ist es, wenn bei der erfindungsgemäßen Verwendung das Phospholipid eine Konzentration an Lyso-Phosphatidylcholin von weniger als 5,6 Gew.% und insbesondere zwischen 5,5 Gew.% und 1,5 Gew.%, an Phosphatidylethanolamin von weniger als 5,2 Gew.% und insbesondere zwischen 5,1 Gew.% und 2,3 Gew.% und an Phosphatidsäure von weniger als 2,9 Gew.% und insbesondere zwischen 2,5 Gew.% und 0,9 Gew.% enthält.

Insbesondere weist das bei der erfindungsgemäßen Verwendung in dem eingesetzten Phospholipid enthaltene Öl freie Fettsäuren, Sterole, Mono- und Diglyceride, Triglyceride, Tocopherol und/oder Fettsäureester auf.

Vorteilhafte Weiterbildungen der erfindungsgemäßen Zusammensetzung sowie der erfindungsgemäßen Verwendung sind in den Unteransprüchen angegeben.

Die erfindungsgemäße Zusammensetzung sowie die erfindungsgemäße Verwendung werden nachfolgend anhand von Ausführungsbeispielen in Verbindung mit den Abbildungen näher erläutert.

quantitative Bestimmung des im Phospholipid enthaltenen Öls

Das jeweils zu untersuchende Phospholipid wird gewogen, in etwa 15 ml bis 20 ml Diethylether gelöst und quantitativ auf eine Chromatografiesäule, wie sie nachfolgend beschrieben ist, unter Verwendung von etwa 130 ml bis 150 ml Diethylether als Eluierungsmittel aufgetrennt. Das gesamte Ethereluat wird in einem zuvor ausgewogenen Rundkolben aufgefangen. Anschließend wird das aufgefangene Diethylether-Eluat unter Vakuum im Rotationsdampfer abdestilliert und der Rundkolben mit dem darin befindlichen Ölen nach Klimatisierung im Normklima zurückgewogen, während die ölfreien Phospholipide in der Säule durch das Adsorbens adsorbiert sind und dort verbleiben. Hieraus errechnet sich der Anteil des in dem Phospholipid enthaltenen Öls wie folgt:

E = Einwaage an phospholipidischer Ausgangssubstanz [g]

LK = Leergewicht des Rundkolbens [g]

R = Rückwaage des Rundkolbens nach Abdestillieren des Diethylethers und Lagerung im Normklima [g]

Ö = prozentualer Ölanteil in der phospholipidischen Ausgangssubstanz

$$\ddot{O} = \frac{R - LK \times 100}{E} \quad [\%]$$

Die zuvor angegebenen Gewichtswerte in Gramm werden auf einer Analysenwaage mit einer Genauigkeit von 0,0001 g bestimmt, wobei die zu analysierende phospholipidische Ausgangssubstanz auf der Analysenwaage in der Größenordnung von etwa 1 g exakt eingewogen wird.

Zur Herstellung der Säule wird wie folgt vorgegangen:

Das als Adsorbens verwendete Kieselgel (Kieselgel 60, 0,063-02 mm, beispielsweise Hersteller: Merck, Artikel-Nr. 7754) wird zunächst auf einen konstanten Wassergehalt von 14,3 Gew.% eingestellt. Hierzu wird vorab der Wassergehalt des jeweils verwendeten Kieselgels mittels Karl-Fischer-Titration bestimmt und die fehlende Menge an Wasser zur Erreichung des Wassergehaltes von 14,3 Gew.% zugesetzt. Von dem so behandelten Kieselgel wird nochmals über eine Karl-Fischer-Titration der Wassergehalt bestimmt, so daß sichergestellt ist, daß das Kieselgel einen Wassergehalt von 14,3 Gew.% aufweist.

30 g des auf einen Wassergehalt von 14,3 Gew.% eingestellten Kieselgels wird in Diethylether aufgeschlämmt und in die Chromatografiesäule eingebracht, die einen Durchmesser von 25 mm aufweist. Der überschüssige Ether wird so weit aus der Säule abgelassen, bis oberhalb des Adsorbens eine etwa 1 cm hohe Etherschicht stehen bleibt. Auf die so vorbereitete Säule wird dann die zu analysierende Probe aufgetragen und aufgetrennt, wie dies eingangs beschrieben ist.

Alle bei dieser Analyse verwendeten Lösungsmittel liegen in der p.a.-Reinheit vor.

Herstellung von Phospholipiden mit unterschiedlichem Ölgehalt

Die zuvor beschriebene analytische und gravimetrische Methode zur Bestimmung des quantitativen Ölanteils wurde dahingehend abgewandelt, daß die dort beschriebene säulenchromatografische Abtrennung des Öls nunmehr als präparative säulenchromatografische Abtrennung angewandt wurde, um die nachfolgend beschriebenen und mit Phospholipid 1 bis 5 bezeichneten Phospholipide herzustellen, die sich in ihrem Ölgehalt unterscheiden.

Hierzu wurde wie folgt vorgegangen:

4.500 g des auf einen Wassergehalt von 14,3 Gew.% eingestellten Kieselgels wird in Diethylether aufgeschlämmt und in eine präparative Chromatografiesäule eingebracht, wobei der Wassergehalt des Kieselgels so eingestellt und überprüft worden ist, wie dies vorstehend bei der quantitativen Bestimmung des Ölgehaltes des Phospholipids beschrieben ist. Der überschüssige Ether wird so weit aus der Säule abgelassen, bis oberhalb des Adsorbens eine etwa 1 cm hohe Etherschicht stehen bleibt. Auf die so vorbereitete Säule wird dann die präparativ aufzutrennende, in etwa 2,5 l Diethylether gelöste Phospholipid-Probe (Einwaage etwa 150 g) aufgetragen und aufgetrennt, wie dies ebenfalls eingangs bei der quantitativen Bestimmung des Öls beschrieben ist. Das aufgefangene Diethylether-Eluat wird unter Vakuum unter Gewinnung des so isolierten Öles abdestilliert. Hiernach konnten etwa 140 g Öl isoliert werden.

Das mit dem ölfreien Phospholipid beladene Adsorbens wurde aus der präparativen Säule entfernt und der Diethylether schonend entfernt.

Das so getrocknete Adsorbens wurde mit einer Mischung aus Chloroform und Methanol (2:1; V:V) mehrfach extrahiert, wobei die Extrakte, die das ölfreie Phospholipid in Lösung enthielten, vereinigt wurden. Nach schonender Abtrennung des Lösungsmittelgemisches wurde das so isolierte trockene Phospholipid unter Ausbildung von fünf, bezüglich des Phospholipids gleichkonzentrierten Proben eingewogen und in Ethanol gelöst. Zu jeder der fünf ethanolischen Phospholipidlösungen wurde das zuvor bei der Auftrennung mit Diethylether gewonnene Öl in den vorgegebenen Mengen (2 Gew.%, 4 Gew.%, 5,8 Gew.%, 7,5 Gew.%, 9 Gew.%) zugesetzt, nachdem dieses Öl zuvor ebenfalls in Ethanol gelöst worden ist. Nach intensivem Vermischen des jeweils ölfreien Phospholipids mit dem Öl wurde das Ethanol unter Ausbildung der nachfolgend quantifizierten Phospholipide 1 bis 5 abgezogen, wobei diese Phospholipide 1 bis 5 zur Herstellung der Ausführungsbeispiele 1 bis 10 verwendet wurde.

Hierbei wiesen diese fünf unterschiedlichen Phospholipidproben folgenden Ölgehalt auf:

Phospholipid 1, Ölgehalt 7,5 Gew.%
Phospholipid 2, Ölgehalt 5,8 Gew.%
Phospholipid 3, Ölgehalt 4 Gew.%
Phospholipid 4, Ölgehalt 2 Gew.% und
Phospholipid 5, Ölgehalt 9 Gew.%.

Bei allen fünf Phospholipiden (Phospholipid 1 bis 5) betrug die Konzentration an
Phosphatidylcholin 76 ± 3 Gew.%,
Lyso-Phosphatidylcholin ≤ 6 Gew.%
Phosphatidylamin ≤ 6 Gew.% und
Phosphatidsäure ≤ 6 Gew.%,
wobei sich diese Angaben jeweils auf das Trockengewicht des Phospholipids beziehen.

Alle Phospholipide wiesen darüber hinaus eine Säurezahl kleiner als 10 und einen Peroxidwert ebenfalls kleiner als 10 auf.

Es wurden die nachfolgenden Ausführungsbeispiele 1 bis 10 unter Verwendung der zuvor beschriebenen Phospholipide 1 bis 5 hergestellt.

Übereinstimmend wiesen die Ausführungsbeispiele 1 bis 5 jeweils als pharmazeutischen Wirkstoff 10 Gew.% Ketoprofen und die Ausführungsbeispiele 6 bis 10 jeweils als pharmazeutischen Wirkstoff 4 Gew.% Diclofenac-Natrium auf.

Darüber hinaus enthielten die Ausführungsbeispiele 1 bis 5 jeweils 59,48 Gew.% Wasser, 10 Gew.% Propylenglykol, 8 Gew.% Isopropanol, 0,25 Gew.% Natriumdihydrogenphosphat,Dihydrat, 0,57 Gew.% Dinatriumphosphat,Dodecahydrat, 1,55 Gew.% Natriumhydroxidlösung und 0,15 Gew.% Pfefferminzöl auf.

Die Ausführungsbeispiele 1 bis 5 unterschieden sich lediglich darin, daß sie zwar identische Mengen, d.h. 10 Gew.%, des zuvor beschriebenen und spezifizierten Phospholipids aufwiesen, wobei es sich bei diesem Phospholipid um die vorstehend aufgeführten Phospholipide 1 bis 5 handelte, die sich in ihrer Ölkonzentration wie folgt unterschieden:

Ausführungsbeispiel 1 enthält 10 Gew.% Phospholipid 1 (Ölgehalt 7,5 Gew.%)
Ausführungsbeispiel 2 enthält 10 Gew.% Phospholipid 2 (Ölgehalt 5,8 Gew.%)
Ausführungsbeispiel 3 enthält 10 Gew.% Phospholipid 3 (Ölgehalt 4 Gew.%)
Ausführungsbeispiel 4 enthält 10 Gew.% Phospholipid 4 (Ölgehalt 2 Gew.%)
Ausführungsbeispiel 5 enthält 10 Gew.% Phospholipid 5 (Ölgehalt 9 Gew.%)

Die zuvor aufgeführten 10 Gew.% der Phospholipide 1 bis 5 wiesen jeweils 7,5 Gew.% Phospholipid 1 bis 5 und 2,5 Gew.% absolutem Ethanol auf.

Darüber hinaus enthielten die Ausführungsbeispiele 6 bis 10 jeweils 56,38 Gew.% Wasser, 15 Gew.% Propylenglykol, 10,25 Gew.% Isopropanol, 0,12 Gew.% Natriumdihydrogenphosphat,Dihydrat, 0,20 Gew.% Pfefferminzöl, 0,66 Gew.% Dinatriumphosphat,Dodecahydrat 0,04 Gew.% Dinatrium-Edetate, 0,02 Gew.% Palmitoylascorbinsäure auf.

Die Ausführungsbeispiele 6 bis 10 unterschieden sich lediglich darin, daß sie zwar identische Mengen, d.h. 13,33 Gew.%, des zuvor beschriebenen Phospholipids aufwiesen, wobei es sich bei diesem Phospholipid um die vorstehend aufgeführten Phospholipide 1 bis 5 handelte, die sich in ihrer Ölkonzentration wie folgt unterschieden:

Ausführungsbeispiel 6 enthält 13,33 Gew.% Phospholipid 1 (Ölgehalt 7,5 Gew.%)
Ausführungsbeispiel 7 enthält 13,33 Gew.% Phospholipid 2 (Ölgehalt 5,8 Gew.%)
Ausführungsbeispiel 8 enthält 13,33 Gew.% Phospholipid 3 (Ölgehalt 4 Gew.%)
Ausführungsbeispiel 9 enthält 13,33 Gew.% Phospholipid 4 (Ölgehalt 2 Gew.%)
Ausführungsbeispiel 10 enthält 13,33 Gew.% Phospholipid 5 (Ölgehalt 9 Gew.%)

Die zuvor aufgeführten 13,33 Gew.% der Phospholipide 1 bis 5 wiesen jeweils 9,998 Gew.% Phospholipid 1 bis 5 und 3,332 Gew.% absolutem Ethanol auf.

Für alle, Ketoprofen als Wirkstoff enthaltenden Ausführungsbeispiele 1 bis 5 wurde ein identisches Herstellungsverfahren verwendet. Hierzu wurde Ketoprofen, Propylenglykol und Isopropanol in einem Mischbehälter vermischt, wobei dieser Mischvorgang unter Stickstoff oder Argon ausgeführt wurde.

Etwa 90 Gew.% der Natriumdihydrogenphosphat-Menge, sowie das Dinatriumphosphat und die Natriumhydroxidlösung wurden miteinander vermischt.

Die zuvor als erstes hergestellte Mischung wurde bei einer Temperatur zwischen 20 °C und 25 °C mit dem jeweiligen Phospholipid (Phospholipid 1 bis 5) versetzt und anschließend so lange gemischt, bis eine klare, gelbliche Lösung resultierte. Zu dieser gelblichen klaren Lösung wurde dann die zuvor als zweites beschriebene vermischte Pufferlösung zugesetzt, wobei die beiden Lösungen solange (unter Argon oder Stickstoff) bei einer Temperatur von maximal 30 °C vermischt wurden, bis eine homogene Lösung resultierte, die mit der restlichen Pufferlösung und dem Pfefferminzöl versetzt wurde und deren pH-Wert auf einen Wert zwischen 7,3 und 7,5 eingestellt wurde.

Für alle, Diclofenac-Natrium als Wirkstoff enthaltenden Ausführungsbeispiele 6 bis 10 wurde ein identisches Herstellungsverfahren verwendet. Hierzu wurde etwa 90 Gew.% des Wassers (gereinigtes Wasser) bei einer Temperatur zwischen 25 °C und 30 °C und einer Drehzahl von 600 Umdrehungen/min mit dem Dinatriumphosphat, Natriumdihydrogenphosphat und dem Natrium-Edetate vermischt. Die so erstellte Lösung wurde auf eine Temperatur auf 20 °C und 25 °C abgekühlt.

In einem zweiten Mischbehälter wurde das Propylenglykol, der Isopropylalkohol und das jeweilige Phospholipid (Phospholipid 1 bis 5) unter vorsichtigem Rühren zu einer homogenen Lösung vermischt. Zu dieser Mischung wurde unter Beibehaltung des Rührens das Ascorbylpalmitat und das Diclofenac-Natrium zugesetzt. Die so erstellte homogene Mischung wurde so lange gemischt, bis eine klare Lösung resultierte, die mit Pfefferminzöl unter kontinuierlichem Rühren versetzt wurde. Nach Messung des pH-Wertes wurde dieser durch Zusatz von Natriumhydroxid-Lösung oder verdünnter Salzsäure auf einen Wert zwischen pH 7,4 - 7,6 eingestellt. Während der Herstellung wurde die Temperatur konstant zwischen 20 °C und 30 °C gehalten.

Permeationsuntersuchungen der zuvor beschriebenen Ausführungsbeispiele 1 bis 10

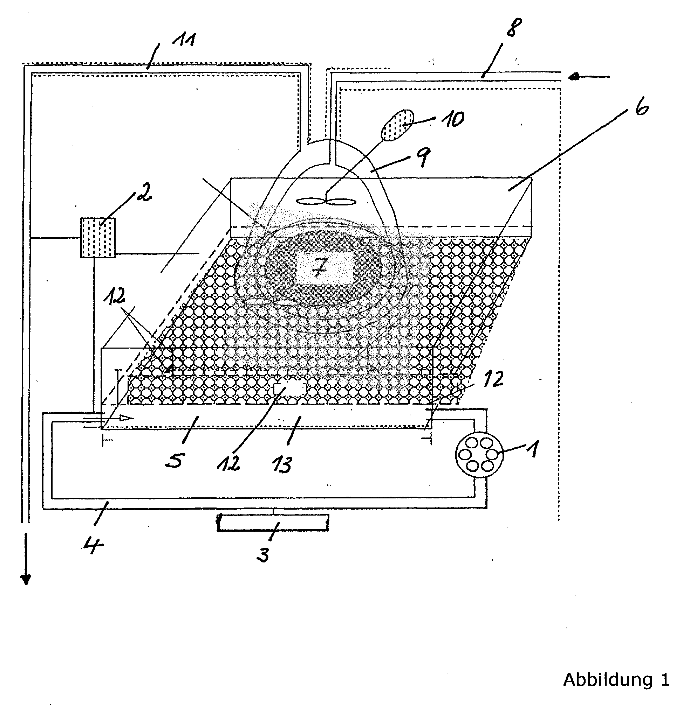

Das nachfolgend beschriebene und in Abbildung 1 gezeigte in-situ-Modell ist insbesondere geeignet zur vergleichenden Bestimmung der Permeationsraten. Diesem Modell liegt die Erfahrung vieler in vivo-Versuche an Jungschweinen und an Probanden zugrunde. Es wurde unter Berücksichtigung möglichst relevanter in-vivo-Bedingungen entwickelt und konnte bereits mehrfach erprobt werden, so daß es mit korrespondierenden in-vivo-Probandenstudien validiert worden ist.

In dem in Abbildung 1 gezeigten Versuchsaufbau zur Bestimmung der Permeation wird das unterhalb der zu untersuchenden Hautprobe und mit einem Metallgitter 12 abgedeckte Akzeptormedium 13 von 90 ml auf 35°C temperiert und kontinuierlich mittels Pumpe 1 mit 1000 ml/h umgepumpt. Die mit dem Akzeptormedium 13 gefüllte Permeationskammer 5 besitzt ein Volumen von 50 ml, das Ausgleichsgefäß 3 zur Probenentnahme und die Schläuche 4 nehmen 40 ml Gesamtvolumen ein. Das Lumen kann mit verschiedenen, hautphysiologischen Lösungen gefüllt werden. Maßgebend für die Wahl des Akzeptormediums ist die Löslichkeit des Wirkstoffes und dessen Nachweis.

Für den Wirkstoff Ketoprofen wurde eine Phosphatpufferlösung pH 7,4 und für den Wirkstoff Diclofenac-Natrium wurde eine Phosphatpufferlösung pH 8,0 ausgewählt. Die Auswahl des Akzeptormediums berücksichtigt die Sinkbedingungen für den jeweiligen Wirkstoff.

Die Kammer 5 wurde blasenfrei gefüllt, damit eine vollständige und gleichmäßige Unterspülung des Gewebes erfolgen konnte. Die Applikationsfläche 7 betrug 28,3 cm$^2$. Die Entnahme von Proben erfolgte intermittierend durch manuelle Entnahme aus dem zirkulierenden Medium und anschließender automatischer Probensammlung für die HPLC. Die Versuchsdauer wurde auf 8 Stunden begrenzt, wobei zu Beginn, nach 30 Minuten, einer Stunde, zwei Stunden, vier Stunden, sechs Stunden und acht Stunden jeweils entsprechende Proben des Akzeptormediums entnommen und analysiert wurden.

Aufgrund der Anordnung des Luftstroms, der Strömungsgeschwindigkeit der eingeleiteten, temperierten Luft und der Verwirbelung auf der Hautfläche wird bei dem verwendeten und in Abbildung 1 gezeigten Versuchsaufbau die Hornschicht nicht hydratisiert. Hierzu diente ein Luftkreislauf, der eine Luftzufuhr von 300 ml Luft/Minute bei 23 °C über die Leitung 8, ein Einführen in eine oberhalb der Applikationsfläche 7 angeordnete Haube 9, innerhalb der ein Ventilator 10 für eine Luftverwirbelung sorgte, und Abluftschläuche 11 zur Abführung der Luft vorsieht.

Die jeweils zu untersuchende Hautprobe wurde auf ein Metallgitter 12 aufgelegt, mit der Haube 9 (ohne Okklusion) abgedeckt, mit dem Luftstrom, der mittels des Ventilators 10 verwirbelte wird, umströmt. Die auf das Metallgitter 12 aufgelegte Hautprobe wird mit der jeweiligen Zusammensetzung beaufschlagt, wobei nachfolgend die Meßmethode zur Bestimmung der Permeation beschrieben ist. Zur Konstanthaltung der Lufttemperatur und der Temperatur des Akzeptormediums dient die nur schematisch mit 2 bezeichnete Temperaturregelung, während eine Haube 6, die die Meßanordnung insbesondere zur Verhinderung von Verschmutzungen, abdeckt.

Die in situ Experimente wurden an excorporierter humaner Bauchhaut von unterschiedlichen Spendern durchgeführt. Die Spender waren zwischen 40 und 50 Jahre alt. Die Untersuchungen wurden an operativ entfernter, abdominaler Humanhaut durchgeführt. Die Größe der zur Verfügung stehenden Hautlappen variierte. Daraus ergab sich die Durchführung von Vierfachversuchen je Humanhautlappen.

Die zur Applikation zur Verfügung stehende Applikationsfläche betrug 28,3 cm². Die Haut wurde frisch entnommen, trocken und bei 4°C gekühlt transportiert. Spätestens nach 2 Stunden wurde der Permeationsversuch begonnen. Die Ganzhaut wurde durch Abschälen des subcutanen Fettgewebes auf einem Schälblock mit Tiefeneinstellung präpariert und auf ihren einwandfreien Zustand mittels Dichtigkeitsprüfung in der Kammer mit Medium durch Druckspülung vor und nach der Untersuchung und mikroskopisch geprüft. Die Hautdicke (ohne Fettgewebe) betrug 1 mm ± 0,1 mm. Das makroskopisch unverletzte und auf Dichtigkeit geprüfte Hautproben wurden direkt in die Versuchsapparatur überführt, wobei die Hautproben auf einem Gitter fixiert und die zur Verfügung stehende Fläche ohne Okklusion konditioniert wurde. Die Konditionierung wurde durch die Regulierung der Vorwärmtemperatur des Mediums und der Temperierung der Luftschläuche sowie durch die Geschwindigkeit des über die Probenfläche streichenden Luftstroms erreicht. Diese Parameter werden innerhalb einer Versuchsreihe konstant gehalten. Die Hautprobe wurde gleichmäßig zirkulierend vom Phosphatpuffer unterspült. Die Temperatur der Hautprobe wurde mit Meßsonden und die Feuchtigkeit mit dem Corneometer überprüft. Die Versorgung der Hautprobe erfolgte über den Phosphatpuffer. Eine mögliche Kontamination der Haut mit dem Desinfektionsmittel Phenylmercuroborat wurde bei der Analytik berücksichtigt.

Die flüssigen Zusammensetzungen wurden mit einem Mikrodispensor gleichmäßig auf der Applikationsfläche von 28,3 cm² verteilt (Rundpistill). Eine Verteilung der aufgetragenen Zusammensetzungen auf der Hautprobe wurde 5 Minuten nach erstem Auftragen nochmals vorgenommen.

Bei Applikation von bis zu 1 g Zusammensetzung pro 28,3 cm² Applikationsfläche war kein Überschuß der jeweiligen Zusammensetzung auf der Oberfläche der Haut im Bereich der Applikationsfläche erkennbar.

Die Permeationsprofile von Ketoprofen und Diclofenac-Natrium wurden durch Entnahme von Doppelproben nach 0, 0,5, 1, 2, 4, 6 und 8 Stunden erstellt. Die Bestimmung des Ketoprofens und des Diclofenac-Natriums erfolgte im Anschluß direkt nach der Entnahme über eine Autosampleranlage. Zur Beurteilung der Permeation des jeweiligen Wirkstoffes wurde dessen Konzentration im Akzeptormedium bestimmt.

Hierzu wurde die Konzentration des Ketoprofens hochdruckflüssigkeitschromatografisch (HPLC-Säule, Xterra RP18 5$\mu$m, 4.6 x 150 mm - Modus: isokratisch - Fluss: 1.0 mL.min-1, Fließmittel: $H_2O/ACN/KH_2PO_4$; Puffer pH 3.5 (55:43:2 (v/v/v)), Puffer: p.a. Chemikalien + deionisiertes Wasser; Fließmittel nicht entgast) unter Verwendung eines UV-Detektors (UV-Detektor Waters Alliance 2795 mit Detektor Waters 2487 dual wavelength 254 nm) bestimmt.

Die Bestimmung der Konzentration des Diclofenac-Natriums erfolgte ebenfalls hochdruckflüssigkeitschromatografisch unter Verwendung eines UV-Detektors (HPLC-System mit folgenden Komponenten: HPLC-Pumpe Merck/Hitachi L-6200, (ternär / Niederdruckgradient), UV-Detektor Merck/Hitachi L-4000, Zweistrahlgerät, Meßbereich 195-380 nm, Integrator Merck/Hitachi D-2500, HPLC-Säule: LiChrospher 100 (Merck), RP18 (5u.m), 250 mm Länge, LiChro-CART-Kartuschensystem, Fließmittel: Methanol/Citratpuffer (3/1); Fließmittel nicht entgast).

Die Ergebnisse der zuvor beschriebenen Permeationsuntersuchungen sind in der nachfolgenden Tabelle 1 und der zugehörigen Abbildung 2 für den Wirkstoff Ketroprofen und in der nachfolgenden Tabelle 2 und der zugehörigen Abbildung 3 für den Wirkstoff Diclofenac-Natrium zusammengefaßt.

Es wurde bei allen Untersuchungen stets dieselbe, vorstehend angegebene Menge der Zusammensetzung auf die Hautprobe aufgetragen, die einer Wirkstoffkonzentration von ca. 25 mg Ketoprofen bzw. von ca. 10 mg Diclofenac-Natrium entsprach. Die wiedergegebenen Werte entsprechen jeweils dem Mittelwert aus einer Vierfachbestimmung.

Tabelle 1

| Permeation von Ketoprofen in das Akzeptormedium Konzentration ($\mu$g) Ketoprofen pro Volumeneinheit (ml) des Akzeptormediums | | | | | |
|---|---|---|---|---|---|
| Ausführungsbeispiel | 1 | 2 | 3 | 4 | 5 |
| Ölkonzentration | 7,5 Gew.% | 5,8 Gew.% | 4,0 Gew.% | 2,0 Gew.% | 9,0 Gew.% |
| | | | | | |
| Entnahmezeit in h | Konzentration in $\mu$g/ml | | | | |
| 0 | 0,000 | 0,000 | 0,000 | 0,000 | 0,000 |
| 0.5 | 0,008 | 0,011 | 0,013 | 0,013 | 0,002 |
| 1 | 0,239 | 0,274 | 0,310 | 0,303 | 0,195 |
| 2 | 2,987 | 3,524 | 4,241 | 3,912 | 2,389 |
| 4 | 8,726 | 10,558 | 11,518 | 11,021 | 7,329 |

(fortgesetzt)

| Entnahmezeit in h | Konzentration in $\mu$g/ml | | | | |
|---|---|---|---|---|---|
| 6 | 15,309 | 18,112 | 21,738 | 19,442 | 12,706 |
| 8 | 17,901 | 21,018 | 24,345 | 22,182 | 14,499 |

Tabelle 2

| Permeation von Diclofenac-Natrium in das Akzeptormedium Konzentration ($\mu$g) Diclofenac-Natrium pro Volumeneinheit (ml) des Akzeptormediums | | | | | |
|---|---|---|---|---|---|
| Ausführungsbeispiel | 6 | 7 | 8 | 9 | 10 |
| Ölkonzentration | 7,5 Gew.% | 5,8 Gew.% | 4,0 Gew.% | 2,0 Gew.% | 9,0 Gew.% |
| | | | | | |
| Entnahmezeit in h | Konzentration in $\mu$g/ml | | | | |
| 0 | 0,000 | 0,000 | 0,000 | 0,000 | 0,000 |
| 0.5 | 0,000 | 0,009 | 0,015 | 0,011 | 0,000 |
| 1 | 0,052 | 0,061 | 0,073 | 0,066 | 0,042 |
| 2 | 0,601 | 0,726 | 1,019 | 0,799 | 0,492 |
| 4 | 1,890 | 2,223 | 2,613 | 2,417 | 1,625 |
| 6 | 3,343 | 4,019 | 4,542 | 4,273 | 2,808 |
| 8 | 3,669 | 4,462 | 5,133 | 4,659 | 3,192 |

**Patentansprüche**

1. Pharmazeutische Zusammensetzung zur Anwendung bei Mensch und Tier, mit mindestens einem systemisch und/oder lokal wirkenden, topisch applizierbaren Wirkstoff und mit mindestens einem Phospholipid, das Phosphatidylcholin in einer Konzentration von wenigstens 60 Gew.%, bezogen auf das Phospholipid, enthält, wobei die Zusammensetzung eine solche flüssige Konsistenz aufweist, daß sie als Tröpfchen versprühbar ist, **dadurch gekennzeichnet, daß** die Zusammensetzung ein solches den Transport des Wirkstoffes durch die Zellmembrane verbesserndes Phospholipid aufweist, das neben wenigstens 60 Gew.% Phosphatidylcholin desweiteren Öl in einer Konzentration zwischen 2 Gew.% und 4,8 Gew.% enthält.

2. Pharmazeutische Zusammensetzung nach Anspruch 1, **dadurch gekennzeichnet, daß** das mindestens eine Phospholipid ein Phospholipidgemisch ist und desweiteren neben Phosphatidylcholin noch Lyso-Phosphatidylcholin, Phosphatidsäure, Phosphatidylethanolamin und/oder Phosphatidylinositol aufweist.

3. Pharmazeutische Zusammensetzung nach Anspruch 1 oder 2, **dadurch gekennzeichnet, daß** das Phospholipid ein aus pflanzlichen Bestandteilen, insbesondere aus Getreidesamen und/oder ölreichen Samen und vorzugsweise aus Sojabohnen oder Sonnenblumen, isoliertes Phospholipid ist.

4. Pharmazeutisches Zusammensetzung nach einem der vorangehenden Ansprüche, **dadurch gekennzeichnet, daß** das Phospholipid eine Konzentration an Phosphatidylcholin von mindestens 75 Gew.% aufweist.

5. Pharmazeutische Zusammensetzung nach einem der vorangehenden Ansprüche, **dadurch gekennzeichnet, daß** das Phospholipid eine Konzentration an Phosphatidylcholin von 78,1 Gew.% $\pm$ 3 Gew.% aufweist.

6. Pharmazeutische Zusammensetzung nach Anspruch 5, **dadurch gekennzeichnet, daß** das Phospholipid eine Konzentration an Lyso-Phosphatidylcholin von weniger als 5,6 Gew.%, an Phosphatidylethanolamin von weniger als 5,2 Gew.% und an Phosphatidsäure von weniger als 2,9 Gew.% enthält.

**7.** Pharmazeutische Zusammensetzung nach Anspruch 6, **dadurch gekennzeichnet, daß** das Phospholipid das Lyso-Phosphatidylcholin in einer Konzentration zwischen 5,5 Gew.% und 1,5 Gew.%, das Phosphatidylethanolamin in einer Konzentration zwischen 5,1 Gew.% und 2,3 Gew.% und die Phosphatidsäure in einer Konzentration zwischen 2,5 Gew.% und 0,9 Gew.% enthält.

**8.** Pharmazeutische Zusammensetzung nach einem der vorangehenden Ansprüche, **dadurch gekennzeichnet, daß** die Zusammensetzung ein anorganisches oder organisches Lösungsmittel aufweist.

**9.** Pharmazeutische Zusammensetzung nach Anspruch 8, **dadurch gekennzeichnet, daß** die Zusammensetzung Wasser und/oder einen Alkohol, insbesondere Ethanol, Isopropanol, ein oder mehrere Glykole und/oder Glycerin, als Lösungsmittel enthält.

**10.** Pharmazeutische Zusammensetzung nach Anspruch 9, **dadurch gekennzeichnet, daß** die Zusammensetzung als Glykol Propylenglykol, Butylenglykol, Pentylenglykol und/oder Hexylenglykol aufweist.

**11.** Pharmazeutische Zusammensetzung nach einem der vorangehenden Ansprüche, **dadurch gekennzeichnet, daß** die Zusammensetzung ein Lösungsmittelgemisch aus Wasser und mindestens einem Alkohol, vorzugsweise Isopropanol, enthält.

**12.** Pharmazeutische Zusammensetzung nach Anspruch 11, **dadurch gekennzeichnet, daß** die Konzentration des Wassers in der flüssigen Zusammensetzung zwischen 50 Gew.% und 95 Gew.%, vorzugsweise zwischen 55 Gew.% und 75 Gew.%, und die Konzentration des mindestens einen Alkohols zwischen 5 Gew.% und 50 Gew.%, vorzugsweise zwischen 8 Gew.% und 25 Gew.%, variiert.

**13.** Pharmazeutische Zusammensetzung nach einem der vorangehenden Ansprüche, **dadurch gekennzeichnet, daß** der mindestens eine Wirkstoff aus der Gruppe ausgewählt ist, die Lokalanästhetika, Antiallergika, Dermatika, Wirkstoffe gegen grippale Infekte und Erkältungskrankheiten, Wirkstoffe zur Behandlung von Neuropathien, Wirkstoffe zur Behandlung von Durchblutungsstörungen, Chemotherapeutika, Chinin, Antimykotika, Antibiotika, Thalidomid, Serotonin, Eicosanoide, Analgetika, Antikonvulsiva, Nicht-steroidale Antirheumatika, Leukotriene, Leukotrienhemmer, Androgene, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren (einzel- und doppelsträngige DNA, einzel- und doppelsträngige RNA, snRNA, DNA-Oligonukleotide, RNA-Oligonukleotide) und Oligopeptide, Antipruriginosa, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Warzen oder Wunden, insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Antiparasitika, Ganglionär angreifende Substanzen, am Symphatikus angreifende Substanzen, am Parasymphatikus angreifende Substanzen, antibakteriell wirkende Pharmaka, Calciumantagonisten, Herz-/Kreislaufmittel, Antiasthmatika, Antitussiva, Expektorantien, Hepatika, Diuretika, Choleretika, Desinfektionsmittel, Spurenelemente, Antiinfektiva, Zytostatika, Antimetaboliten, Hormonantagonisten, Immunmodulatoren sowie Salze der zuvor genannten Wirkstoffe umfaßt.

**14.** Pharmazeutische Zusammensetzung nach Anspruch 13, **dadurch gekennzeichnet, daß** das Analgetikum aus der Gruppe ausgewählt ist, die Diclofenac, Ketoprofen und Ibuprofen umfaßt.

**15.** Pharmazeutische Zusammensetzung nach Anspruch 13 oder 14, **dadurch gekennzeichnet, daß** das Analgetikum Diclofenac ist und daß das Diclofenac in der Zusammensetzung als Alkalisalz, insbesondere als Natriumsalz, vorliegt.

**16.** Pharmazeutische Zusammensetzung nach einem der Ansprüche 13 bis 15, **dadurch gekennzeichnet, daß** das Diclofenac in einer Konzentration zwischen 0,1 Gew.% und 10 Gew.%, vorzugsweise in einer Konzentration zwischen 1 Gew.% und 6 Gew.%, in der Zusammensetzung enthalten ist.

**17.** Pharmazeutische Zusammensetzung nach Anspruch 13 oder 14, **dadurch gekennzeichnet, daß** die Zusammensetzung als Analgetikum Ketoprofen enthält und daß die Konzentration des Ketoprofens in der Zusammensetzung zwischen 5 Gew.% und 15 Gew.%, vorzugsweise zwischen 8 Gew.% und 12 Gew.%, variiert.

**18.** Pharmazeutische Zusammensetzung nach einem der vorangehenden Ansprüche, **dadurch gekennzeichnet, daß** die Zusammensetzung das mindestens eine Phospholipid in einer Konzentration zwischen 0,5 Gew.% und 20 Gew.%, vorzugsweise in einer Konzentration zwischen 5 Gew.% und 13 Gew.%, aufweist.

19. Pharmazeutische Zusammensetzung nach einem der vorangehenden Ansprüche, **dadurch gekennzeichnet, daß** die Zusammensetzung desweiteren mindestens einen Komplexbilder, insbesondere Ethylentetraessigsäure, mindestens eine Puffersubstanz, insbesondere einen Phosphatpuffer, mindestens ein Antioxidationsmittel, insbesondere Palmitoylascorbinsäure, mindestens einen Duftstoff und/oder ein Aroma aufweist.

20. Pharmazeutische Zusammensetzung nach einem der vorangehenden Ansprüche, **dadurch gekennzeichnet, daß** das in dem mindestens einem Phospholipid enthaltene Öl freie Fettsäuren, Sterole, Mono- und Diglyceride, Triglyceride, Tocopherol und/oder Fettsäureester aufweist.

21. Verwendung von mindestens einem Phospholipid als Permeations- und/oder Penetrationsbeschleuniger in einer pharmazeutischen Zusammensetzung nach einem der vorangehenden Ansprüche, **dadurch gekennzeichnet, daß** das Phospholipid Phosphatidylcholin in einer Konzentration von wenigstens 60 Gew.%, bezogen auf das Phospholipid, enthält und daß das Phospholipid neben den wenigstens 60 Gew.% Phosphatidylcholin desweiteren Öl in einer Konzentration zwischen 4,8 Gew.% und 2 Gew.%, enthält.

22. Verwendung nach Anspruch 21, **dadurch gekennzeichnet, daß** das Phospholipid desweiteren Öl in einer Konzentration zwischen 4 Gew.% und 2 Gew.% enthält.

23. Verwendung nach Anspruch 21 oder 22, **dadurch gekennzeichnet, daß** das Phospholipid ein Phospholipidgemisch ist und desweiteren neben Phosphatidylcholin noch Lyso-Phosphatidylcholin, Phosphatidsäure, Phosphatidylethanolamin und/oder Phosphatidylinositol aufweist.

24. Verwendung nach einem der Ansprüche 21 bis 23, **dadurch gekennzeichnet, daß** das Phospholipid ein aus pflanzlichen Bestandteilen, insbesondere aus Getreidesamen und/oder ölreichen Samen und vorzugsweise aus Sojabohnen oder Sonnenblumen, isoliertes Phospholipid ist.

25. Verwendung nach einem der Ansprüche 21 bis 24, **dadurch gekennzeichnet, daß** das Phospholipid eine Konzentration an Phosphatidylcholin von mindestens 75 Gew.% aufweist.

26. Verwendung nach Anspruch 25, **dadurch gekennzeichnet, daß** das Phospholipid eine Konzentration an Phosphatidylcholin von 78,1 Gew.% $\pm$ 3 Gew.% aufweist.

27. Verwendung nach einem der Ansprüche 21 bis 26, **dadurch gekennzeichnet, daß** das Phospholipid eine Konzentration an Lyso-Phosphatidylcholin von weniger als 5,6 Gew.%, an Phosphatidylethanolamin von weniger als 5,2 Gew.% und an Phosphatidsäure von weniger als 2,9 Gew.% enthält.

28. Verwendung nach einem der Ansprüche 21 bis 27, **dadurch gekennzeichnet, daß** das Phospholipid das Lyso-Phosphatidylcholin in einer Konzentration zwischen 5,5 Gew.% und 1,5 Gew.%, das Phosphatidylethanolamin in einer Konzentration zwischen 5,1 Gew.% und 2,3 Gew.% und die Phosphatidsäure in einer Konzentration zwischen 2,5 Gew.% und 0,9 Gew.% enthält.

29. Verwendung nach einem der Ansprüche 21 bis 28, **dadurch gekennzeichnet, daß** das in dem mindestens einem Phospholipid enthaltene Öl freie Fettsäuren, Sterole, Mono- und Diglyceride, Triglyceride, Tocopherol und/oder Fettsäuren aufweist.

**Claims**

1. A pharmaceutical composition for application in human and animals, with at least one systemically and/or locally acting, topically applicable active ingredient and with at least one phospholipid, which contains phosphatidylcholine in a concentration of at least 60 % by weight, referring to the phospholipid, whereby the composition has such a liquid consistency, that it is able to be sprayed as droplets, **characterized in that** the composition contains such a phospholipid, which improves the transport of the active ingredient trough the cell membrane and which additionally comprises oil in a concentration of between 2 % by weight and 4,8 % by weight besides the at least 60 % by weight phosphatidylcholine.

2. The pharmaceutical composition according to claim 1, **characterized in that** the phospholipid is a phospholipid mixture and furthermore contains lyso-phosphatidylcholine, phosphatidic acid, phosphatidylethanolamine and /or phosphatidylinositol besides phosphatidylcholine.

3. The pharmaceutical composition according to claim 1 or 2, **characterized in that** the phospholipid is a phospholipid, which is isolated from vegetable components, especially from grain seeds and/or oil-rich seeds and preferably from soy beans or from sunflowers.

4. The pharmaceutical composition according to one of the preceding claims, **characterized in that** the phospholipid contains a concentration of phosphatidylcholine of at least 75 % by weight.

5. The pharmaceutical composition according to one of the preceding claims, **characterized in that** the phospholipid contains a concentration of phosphatidylcholine of 78,1 % by weight $\pm$ 3 % by weight.

6. The pharmaceutical composition according to claim 5, **characterized in that** the phospholipid contains a concentration of lysophosphatidylcholine of less than 5,6 % by weight, of phosphatidylethanolamine of less than 5,2 % by weight and of phosphatidic acid of less than 2,9 % by weight.

7. The pharmaceutical composition according to claim 6, **characterized in that** the phospholipid contains the lyso-phosphatidylcholine in a concentration between 5,5 % by weight and 1,5 % by weight, the phosphatidylethanolamine in a concentration between 5,1 % by weight and 2,3 % by weight and the phosphatidic acid in a concentration between 2,5 % by weight and 0,9 % by weight.

8. The pharmaceutical composition according to one of the preceding claims, **characterized in that** the composition contains an inorganic or organic solvent.

9. The pharmaceutical composition according to claim 8, **characterized in that** the composition contains water and/or an alcohol, especially ethanol, isopropyl alcohol, one or several glycols and/or glycerol as solvent.

10. The pharmaceutical composition according to claim 9, **characterized in that** the composition contains propylene glycol, butylene glycol, pentylene glycol, and/or hexylene glycol as glycol.

11. The pharmaceutical composition according to one of the preceding claims, **characterized in that** the composition contains a solvent mixture comprising water and at least one alcohol, preferably isopropyl alcohol.

12. The pharmaceutical composition according to claim 11, **characterized in that** the concentration of water in the liquid composition varies between 50 % by weight and 95 % by weight, preferably between 55 % by weight and 75 % by weight, and that the concentration of the at least one alcohol varies between 5 % by weight and 50 % by weight, preferably between 8 % by weight and 25 % by weight.

13. The pharmaceutical composition according to one of the preceding claims, **characterized in that** the at least one active ingredient is chosen from the group, including local anesthetics, anti-allergic agents, dermatics, active ingredients against flu infections and colds, active ingredients for the treatment of neuropathies, active ingredients for the treatment of disturbed circulation, chemotherapy drugs, quinine, antimycotics, antibiotics, thalidomide, serotonin, eicosanoids, analgesics, anticonvulsants, nonsteroidal antirrheumatics, leukotrienes, leukotriene inhibitors, androgens, antiandrogens, corticoids, opiate receptor antagonists, blood clotting inhibitory substances, thrombocyte aggregation inhibitors, histamine antagonists, regulatory and enzymatically acting peptides and proteins, nucleic acids (single and double-stranded DNA, single and double-stranded RNA, snRNA, DNA oligonucleotides, RNA oligonucleotides) and oligopeptides, antipruritics, antidiabetics, prostaglandins, prostaglandin synthesis inhibitors, antiviral-acting or virostatic-acting substances, antimicrobial-acting substances, active ingredients against prions, immune suppressants, hormones, active ingredients for treatment of warts or wounds, especially chronic wounds, vitamins, plant extracts or essences of plant extracts, psychoactive drugs, active ingredients which influences sleep, analeptics, general anesthetics, muscle relaxants, antiepileptics, antiparkinson agents, antiemetics, antiparasitics, ganglion-active ingredients, sympathetic-active ingredients, parasympathetic-active ingredients, antibacterial-acting drugs, calcium antagonists, cardiovascular agents, antiasthmatics, antitussives, expectorants, hepatics, diuretics, choleretics, disinfectants, trace elements, antiinfectives, cytostatics, antimetabolites, hormone antagonists, immune modulators, as well as derivates and salts of the aforementioned active ingredients.

**14.** The pharmaceutical composition according to claim 13, **characterized in that** the analgesic is chosen from the group including diclofenac, ketoprofen and ibuprofen.

**15.** The pharmaceutical composition according to claim 13 or 14, **characterized in that** in the composition the analgesic is diclofenac and the diclofenac is present in the composition as alkaline salt, especially as sodium salt.

**16.** The pharmaceutical composition according to one of the claims 13 to 15, **characterized in that** the diclofenac is contained in the composition in a concentration between 0,1 % by weight and 10 % by weight, preferably in a concentration between 1 % by weight and 6 % by weight.

**17.** The pharmaceutical composition according to claim 13 or 14, **characterized in that** the composition contains ketoprofen as at least one active ingredient and that the concentration of the ketoprofen in the composition varies between 5 % by weight and 15 % by weight, preferably between 8 % by weight and 12 % by weight.

**18.** The pharmaceutical composition according to one of the preceding claims, **characterized in that** the composition comprises at least one phospholipid in a concentration between 0,5 % by weight and 20 % by weight, preferably in a concentration between 5 % by weight and 13 % by weight.

**19.** The pharmaceutical composition according to one of the preceding claims, **characterized in that** the composition furthermore contains a complexing agent, especially ethylene tetra acetic acid, at least one buffering agent, especially a phosphate buffer, at least one antioxidant, especially palmitoyl ascorbic acid, at least one perfume and/or an aroma.

**20.** The pharmaceutical composition according to one of the preceding claims, **characterized in that** the oil contained in the at least one phospholipid comprises free fatty acids, sterols, mono- and diglycerides, triglycerides, tocopherol and/or fatty acid esters.

**21.** Use of at least one phospholipid as permeation and/or penetration enhancer in a pharmaceutical composition according to one of the preceding claims, **characterized in that** the phopholipid contains phosphatidylcholine in a concentration of at least 60 % by weight, relative to the phospholipid and that the phospholipid besides the at least 60 % by weight phosphatidylcholine further comprises oil in a concentration between 4,8 % by weight and 2 % by weight.

**22.** The use according to claim 21, **characterized in that** the phospholipid further contains oil in a concentration between 4 % by weight and 2 % by weight.

**23.** The use according to claim 21 or 22, **characterized in that** the phospholipid is a mixture of phospholipids and furthermore contains lyso-phosphatidylcholine, phosphatidic acid, phosphatidylethanolamine and/or phosphatidylinositol besides phosphatidylcholine.

**24.** The use according to one of the claims 21 to 23, **characterized in that** the phospholipid is a phospholipid, which is isolated from vegetable components, especially from grain seeds and/or oil-rich seeds and preferably from soy beans or from sunflowers.

**25.** The use according to one of the claims 21 to 24, **characterized in that** the phospholipid contains a concentration of phosphatidylcholine of at least 75 % by weight.

**26.** The use according to claim 25, **characterized in that** the phospholipid contains a concentration of phosphatidylcholine of 78,1 % by weight $\pm$ 3 % by weight.

**27.** The use according to one of the claims 21 to 26, **characterized in that** the phospholipid contains a concentration of lyso-phosphatidylcholine of less than 5,6 % by weight, of phosphatidylethanolamine of less than 5,2 % by weight and of phosphatidic acid of less than 2,9 % by weight.

**28.** The use according to one of the claims 21 to 27, **characterized in that** the phospholipid contains the lyso-phosphatidylcholine in a concentration between 5,5 % by weight and 1,5 % by weight, the phosphatidylethanolamine in a concentration between 5,1 % by weight and 2,3 % by weight and the phosphatidic acid in a concentration between 2,5 % by weight and 0,9 % by weight.

**29.** The use according to one of the claims 21 to 28, **characterized in that** the oil contained in the at least one phospholipid comprises free fatty acids, sterols, mono- and diglycerides, triglycerides, tocopherol and/or fatty acids.

**Revendications**

**1.** Composition pharmaceutique à utiliser chez l'homme et les animaux, comprenant au moins un principe actif à action systémique et/ou locale, à application topique et comprenant au moins un phospholipide, qui contient de la phosphatidylcholine en une concentration d'au moins 60 % en poids par rapport au phospholipide, dans laquelle la composition présente une consistance liquide telle qu'elle peut être pulvérisée sous la forme de gouttelettes, **caractérisée en ce que** la composition présente un phopholipide du type améliorant le transport du principe actif à travers les membranes cellulaires, lequel contient, outre au moins 60 % en poids de phosphatidylcholine, par ailleurs de l'huile en une concentration comprise entre 2 % en poids et 4,8 % en poids.

**2.** Composition pharmaceutique selon la revendication 1, **caractérisée en ce que** l'au moins un phospholipide est un mélange de phospholipides et présente, par ailleurs, outre la phosphatidylcholine, également de la lyso- phosphatidylcholine, de l'acide phosphatidique, de la phosphatidyléthanolamine et/ou du phosphatidylinositol.

**3.** Composition pharmaceutique selon la revendication 1 ou 2, **caractérisée en ce que** le phospholipide est un phospholipide isolé d'éléments végétaux, en particulier de graines de céréales et/ou de graines riches en huile et de préférence de fèves de soja ou de tournesols.

**4.** Composition pharmaceutique selon l'une quelconque des revendications précédentes, **caractérisée en ce que** le phospholipide présente une concentration en phosphatidylcholine d'au moins 75 % en poids.

**5.** Composition pharmaceutique selon l'une quelconque des revendications précédentes, **caractérisée en ce que** le phospholipide présente une concentration en phosphatidylcholine de 78,1 % en poids $\pm$ 3 % en poids.

**6.** Composition pharmaceutique selon la revendication 5, **caractérisée en ce que** le phospholipide contient une concentration en lyso- phosphatidylcholine inférieure à 5,6 % en poids, en phosphatidyléthanolamine inférieure à 5,2 % en poids et en acide phosphatidique inférieure à 2,9 % en poids.

**7.** Composition pharmaceutique selon la revendication 6, **caractérisée en ce que** le phospholipide contient la lyso-phosphatidylcholine en une concentration comprise entre 5,5 % en poids et 1,5 % en poids, la phosphatidyléthanolamine en une concentration comprise entre 5,1 % en poids et 2,3 % en poids et l'acide phosphatidique en une concentration comprise entre 2,5 % en poids et 0,9 % en poids.

**8.** Composition pharmaceutique selon l'une quelconque des revendications précédentes, **caractérisée en ce que** la composition présente un solvant inorganique ou organique.

**9.** Composition pharmaceutique selon la revendication 8, **caractérisée en ce que** la composition contient en tant que solvants de l'eau et/ou un alcool, en particulier de l'éthanol, de l'isopropanol, un ou plusieurs glycols et/ou de la glycérine.

**10.** Composition pharmaceutique selon la revendication 9, **caractérisée en ce que** la composition présente en tant que glycol du propylène glycol, du butylène glycol, du pentylène glycol et/ou de l'hexylène glycol.

**11.** Composition pharmaceutique selon l'une quelconque des revendications précédentes, **caractérisée en ce que** la composition contient un mélange de solvants composé d'eau et d'au moins un alcool, de préférence de l'isopropanol.

**12.** Composition pharmaceutique selon la revendication 11, **caractérisée en ce que** la concentration de l'eau dans la composition liquide varie entre 50 % en poids et 95 % en poids, de préférence entre 55 % en poids et 75 % en poids, et **en ce que** la concentration de l'au moins un alcool varie entre 5 % en poids et 50 % en poids, de préférence entre 8 % en poids et 25 % en poids.

**13.** Composition pharmaceutique selon l'une quelconque des revendications précédentes, **caractérisée en ce que** l'au moins un principe actif est choisi parmi le groupe qui comprend les anesthésiques locaux, les antiallergiques, les produits dermatologiques, les principes actifs contre les infections grippales et les rhumes, les principes actifs servant à traiter des neuropathies, les principes actifs servant à traiter des troubles de la circulation, des agents chimiothérapeutiques, de la quinine, des antifongiques, des antibiotiques, de la thalidomide, de la sérotonine, des éicosanoïdes, des analgésiques, des anticonvulsifs, des anti-rhumatismaux non-stéroïdiens, des leucotriènes, des inhibiteurs de leucotriène, des androgènes, des anti-androgènes, des corticoïdes, des antagonistes de récepteur d'opiacé, des substances anticoagulantes, des antiagrégants plaquettaires, des antagonistes d'histamine, des peptides et des protéines à action régulatrice et enzymatique, des acides nucléiques (ADN monobrin et à double brin, ARN monobrin et à double brin, snARN, des oligonucléotides d'ADN, des oligonucléotides d'ARN) et des oligopeptides, des substances antiprurigineuses, des antidiabétiques, des prostaglandines, des inhibiteurs de synthèse de prostaglandine, des substances ayant une action antivirale ou virostatique, des substances ayant une action antimicrobienne, des principes actifs contre des prions, des immunodépresseurs, des hormones, des principes actifs servant à traiter des verrues ou des plaies, en particulier des plaies chroniques, des vitamines, des extraits de plantes ou extraits composés d'extraits de plantes, des psychotropes, des principes actifs ayant un effet sur le sommeil, des analeptiques, des anesthésiques généraux, des décontractants musculaires, des antiépileptiques, des médicaments antiparkinsoniens, des antiémétiques, des antiparasitaires, des substances à attaque ganglionnaire, des substances attaquant le sympathique, des substances attaquant le parasympathique, des produits pharmaceutiques à action antibactérienne, des antagonistes du calcium, des médicaments pour le coeur/cardiovasculaires, des antiasthmatiques, des antitussifs, des expectorants, des remèdes hépatiques, des diurétiques, des produits cholérétiques, des produits désinfectants, des oligo-éléments, des anti-infectieux, des cytostatiques, des antimétabolites, des antagonistes d'hormone, des immunomodulateurs ainsi que des sels des principes actifs mentionnés plus haut.

14. Composition pharmaceutique selon la revendication 13, **caractérisée en ce que** l'analgésique est choisi parmi le groupe, qui comprend le diclofénac, le kétoprofène et l'ibuprofène.

15. Composition pharmaceutique selon la revendication 13 ou 14, **caractérisée en ce que** l'analgésique est le diclofénac, et **en ce que** le diclofénac est présent dans la composition sous la forme d'un sel alcalin, en particulier d'un sel de sodium.

16. Composition pharmaceutique selon l'une quelconque des revendications 13 à 15, **caractérisée en ce que** le diclofénac est contenu dans la composition en une concentration comprise entre 0,1 % en poids et 10 % en poids, de préférence en une concentration comprise entre 1 % en poids et 6 % en poids.

17. Composition pharmaceutique selon la revendication 13 ou 14, **caractérisée en ce que** la composition contient en tant qu'analgésique du kétoprofène, et **en ce que** la concentration de kétoprofène dans la composition varie entre 5 % en poids et 15 % en poids, de préférence entre 8 % en poids et 12 % en poids.

18. Composition pharmaceutique selon l'une quelconque des revendications précédentes, **caractérisée en ce que** la composition présente l'au moins un phospholipide en une concentration comprise entre 0,5 % en poids et 20 % en poids, de préférence en une concentration comprise entre 5 % en poids et 13 % en poids.

19. Composition pharmaceutique selon l'une quelconque des revendications précédentes, **caractérisée en ce que** la composition présente par ailleurs au moins un agent complexant, en particulier un acide éthylène tétraacétique, au moins une substance tampon, en particulier un tampon de phosphate, au moins un agent antioxydant, en particulier un acide palmitoyle-ascorbique, au moins un parfum et/ou un arôme.

20. Composition pharmaceutique selon l'une quelconque des revendications précédentes, **caractérisée en ce que** l'huile contenue dans l'au moins un phospholipide présente des acides gras libres, des stérols, des mono- et diglycérides, des triglycérides, du tocophérol et/ou des esters d'acide gras.

21. Utilisation d'au moins un phospholipide en tant qu'activateur de perméation et/ou de pénétration dans une composition pharmaceutique selon l'une quelconque des revendications précédentes, **caractérisée en ce que** le phospholipide contient de la phosphatidylcholine en une concentration d'au moins 60 % en poids, par rapport au phospholipide, et **en ce que** le phospholipide contient, outre les au moins 60 % en poids de phosphatidylcholine, par ailleurs de l'huile en une concentration comprise entre 4,8 % en poids et 2 % en poids.

22. Utilisation selon la revendication 21, **caractérisée en ce que** le phospholipide contient par ailleurs de l'huile en une concentration comprise entre 4 % en poids et 2 % en poids.

**23.** Utilisation selon la revendication 21 ou 22, **caractérisée en ce que** le phospholipide est un mélange de phospholipides et présente par ailleurs, outre la phosphatidylcholine ou la lyso-phosphatidylcholine, de l'acide phosphatidique, de la phosphatidyléthanolamine et/ou du phosphatidylinositol.

**24.** Utilisation selon l'une quelconque des revendications 21 à 23, **caractérisée en ce que** le phospholipide est un phospholipide isolé d'éléments végétaux, en particulier de graines de céréales et/ou de graines riches en huile et de préférence de fèves de soja ou de tournesols.

**25.** Utilisation selon l'une quelconque des revendications 21 à 24, **caractérisée en ce que** le phospholipide présente une concentration en phosphatidylcholine d'au moins 75 % en poids.

**26.** Utilisation selon la revendication 25, **caractérisée en ce que** le phospholipide présente une concentration en phosphatidylcholine de 78,1 % en poids $\pm$ 3 % en poids.

**27.** Utilisation selon l'une quelconque des revendications 21 à 26, **caractérisée en ce que** le phospholipide contient une concentration en lyso-phosphatidylcholine inférieure à 5,6 % en poids, en phosphatidyléthanolamine inférieure à 5,2 % en poids et en acide phosphatidique inférieure à 2,9 % en poids.

**28.** Utilisation selon l'une quelconque des revendications 21 à 27, **caractérisée en ce que** le phospholipide contient la lyso- phosphatidylcholine en une concentration comprise entre 5,5 % en poids et 1,5 % en poids, la phosphatidyléthanolamine en une concentration comprise entre 5,1 % en poids et 2,3 % en poids et l'acide phosphatidique en une concentration comprise entre 2,5 % en poids et 0,9 % en poids.

**29.** Utilisation selon l'une quelconque des revendications 21 à 28, **caractérisée en ce que** l'huile contenue dans l'au moins un phospholipide présente des acides gras libres, des stérols, des mono- et diglycérides, des triglycérides, du tocophérol et/ou des esters d'acide gras.

Abbildung 1

In-situ Permeation von Ketoprofen durch die Haut, abhängig von der Konzentration des im Phospholipid enthaltenen Öles

Legend:
- P714/1 oil 7.5%
- P714/2 oil 5.8%
- P714/3 oil 4.0%
- P714/4 oil 2.0%
- P714/5 oil 9.0%

Y-axis: µg/µl
X-axis: Stunden

Abbildung 2

EP 2 638 894 B1

In-situ Permeation von Diclofenac-Natrium durch die Haut, abhängig von der Konzentration des im Phospholipid enthaltenen Öles

—▲— P324/1 oil 7.5%
—✕— P324/2 oil 5.8%
—✳— P324/3 oil 4.0%
—◆— P324/4 oil 2.0%
—▦— P324/5 oil 9.0%

Stunden

Abbildung 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0704206 A
- DE 102010027315
- DE 102010027315 A
- DE 102010027315 A1
- EP 0521398 A2

**In der Beschreibung aufgeführte Nicht-Patentliteratur**

- **ZHENG GUO et al.** Enzymatic modification of phospholipids for functional applications and human nutrition. *Biotechnology Advances,* 2005, vol. 23, 203-259